# EUROPEAN PATENT SPECIFICATION

(11) **EP 0 805 664 B1**
(45) Date of publication and mention of the grant of the patent: **12.04.2000**
(21) Application number: 95942369.0
(22) Date of filing: 22.12.1995
(51) Int. Cl.: A61F 13/15

(54) **A METHOD OF SECURING ELASTIC NON-WOVEN**
VERFAHREN ZUR HERSTELLUNG NICHT-GEWEBTER ELASTISCHER ARTIKEL
PROCEDE D'OBTENTION DE NON TISSES ELASTIQUES

(30) Priority: 27.12.1994 SE 9404524
(43) Date of publication of application: 12.11.1997
(73) Proprietor: SCA Hygiene Products AB, 405 03 Göteborg (SE)
(72) Inventor: PERNEBORN, Robert, S-416 74 Gothenburg (SE); JOHANSSON, Dan, S-426 52 Västra Frölunda (SE); NILSSON, Jörgen, S-411 29 Gothenburg (SE)
(74) Representative: Kierkegaard, Lars-Olov
(86) International application number: SE9501579
(87) International publication number: WO9619958

(56) References cited:
- DE-C- 2 649 948
- GB-A- 2 213 701
- US-A- 4 626 184

## Description

### BACKGROUND

The present invention relates to a method and an apparatus according preamble of claims 1 and 10 respectively for producing an absorbent structure in an absorbent article, such as a sanitary napkin, panty guard, incontinence guard, diaper or like article which are manufactured with the aid of an air-permeable forming element, a so-called wire or a mat-forming wheel, which includes moulds or forms, and in which manufacture one or more elastic threads are mounted on a non-woven material in a stretched state, or in which non-woven material which is in itself elastic is used.

Such a method and such an apparatus are known from the document DE-A-2649948, which represents the preamble of claim 1 and claim 10.

### TECHNICAL BACKGROUND

A number of different designs of absorbent articles of this kind are known to the art. The absorbent body of such products is produced conventionally by dry-defibering cellulose pulp, for instance in reel, bale or sheet form, and converting the fluffed pulp to a pulp mat, sometimes while admixing so-called superabsorbents with the pulp, such absorbents being polymers that are capable of absorbing several times their own weight of water or body fluid. The pulp core is often compressed, partly to enhance its liquid-dispersion ability, and partly to reduce the bulk of the pulp core and therewith obtain a product which is as compact as possible. The absorbent body may also include other constituents, for instance constituents which will enhance its liquid-dispersion ability and its ability to resist deformation in use. Examples of such constituents are cellulose fibres which have been stiffened chemically or in some other way. Other examples of such constituents are different types of binding fibres. In the technical field concerned here, fibre material is deposited on a so-called wire, i.e. some type of air-permeable conveyor belt with the aid of a subpressure, by delivering a mixture of air and absorbent material to the conveyor belt, wherewith the air flows through the air-permeable wire surfaces and leaves absorbent body material carried by the air on said surfaces. Because the layers of deposited material are in themselves air-permeable, layers of material can be deposited successively in this way, until the absorbent body is formed.

U.S. 4,675,144 teaches an arrangement for producing the aforesaid absorbent bodies, comprising forming a fibre web on an air-permeable endless belt, a so-called wire, as before mentioned. Absorbent bodies are then cut-out from the thus formed fibre web.

When manufacturing absorbent articles in accordance with the aforesaid method, it is normal to feed an air-permeable non-woven material to the wire such as to form an underlay for the fibre material deposited on the wire. This non-woven material may be elastic and will later form the sheet that lies proximal to the wearer of the article.

### TECHNICAL PROBLEM

One problem associated with the manufacture of absorbent articles with the aid of an air-permeable wire or a mat-forming wheel, especially when an elastic casing material is used, is that it is necessary to hold the elastic casing material stretched while depositing air-carried material on a wire when a wire is used or until the moulds on the mat-forming wheel are filled and until remaining elements of the article have been applied. Thus, certain parts of the prospective product, preferably the edges thereof, must be kept reserved for securing devices, which means that the elastic casing material is not utilized to the full, among other things. The securing devices also place certain demands on the tensile strength of the material. In the case of present-day techniques, pleats or folds are created when function and tensile strength is reduced or impaired, i.e. the non-woven material is gathered together and wrinkled.

### DISCLOSURE OF THE INVENTION

The problem of holding the preferably elastic casing material, preferably non-woven material, stretched while manufacturing the absorbent article is solved by the present invention, by transferring the elastic casing material in a stretched state to the forming element which is covered with a preferably air-permeable covering which has a coefficient of friction such that the preferably elastic casing material will be held in a stretched state on the forming element by the frictional forces thus generated, possibly in coaction with the prevailing subpressure. The present invention also enables the article to be loosened effectively from the forming element when the subpressure ceases. The components involved in the manufacture of the article, such as conveyor belts, rollers, rolls, etc., may also be covered with said material so as to therewith avoid deformation thereof. This obviates the need for the covered components to be air-permeable and subjected to subpressure.

Other particular embodiments of the invention are set out in claims 2-9, 11-18.

### BRIEF DESCRIPTION OF THE DRAWING

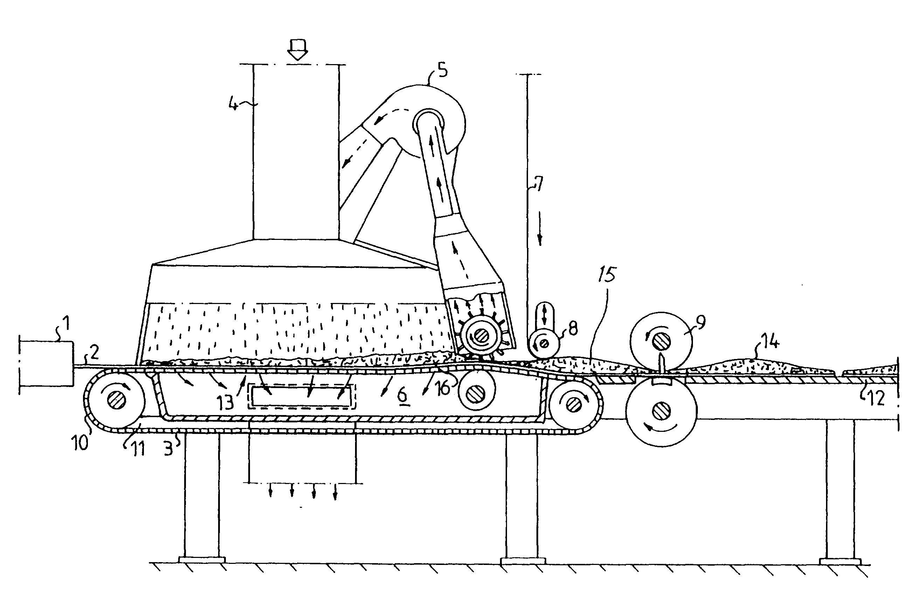

Fig. 1 illustrates an exemplifying embodiment of a mat-former that can be used when applying the method according to the invention.

### DESCRIPTION OF AN EXEMPLIFYING EMBODIMENT

The invention is based on the coaction of a first material having a high coefficient of friction with a second material which shall be held firmly on the first material, and optionally a subpressure which holds the second material firmly on the first material in accordance with the preferred embodiment. The holding effect thus obtained is sufficiently great to counteract the contraction force exerted by the elastic in said second material, i.e. in the elastic, preferably air-permeable casing material which is applied to the first material in a stretched state. In the case of the preferred embodiment, the first material having a high coefficient of friction and with which the forming element is covered is comprised of a textile material having standing fibres and mounted on the forming element, in the preferred case a wire, so that it can be easily exchanged. In the case of the preferred embodiment, which is shown in Fig. 1, the casing material is comprised of an elastic non-woven material 2, said casing material being the material that is intended to lie proximal to the wearer's body when the finished product is used. The material 2 is stretched by the mechanical device 1 prior to being transferred to the wire 3 covered with material 10 having a high coefficient of friction, where the material 2 is held firmly in its stretched state by friction in coaction with the air stream through the covered wire 3. This air stream is generated by the subpressure chamber 6 placed beneath the wire take-up zone 13 on which the absorbent-body material 16 is deposited. The stretched elastic non-woven material 2 is successively overlaid with layers of material forming the absorbent body 15, this material being delivered via the mat-forming hood 4 and being drawn firmly by suction by the aforesaid subpressure prevailing in the chamber 6. Surplus material is removed by means of the device 5 and the absorbent body 15 is given its shape and extension thickness-wise. The removed material is returned to the mat-forming hood 4, optionally via a homogenizer to avoid the formation of flocks by said material. The rear side layer 7 of the product is joined to the elastic non-woven material 2 with the material deposited thereon with the aid of the device 8, whereafter the products are conveyed to a cutting/end-sealing device 9.

Subsequent to treatment by the device 9, the individual products 14 are conveyed on a conveyor belt 12, possibly for further treatment and packaging. In order to prevent the air-permeability of the covered wire 3 being impaired as a result of clogging, there is generated in the space 11 an overpressure which functions to continuously blow clean the air-permeating openings in the wire 3 and the material 10 having a high coefficient of friction deposited thereon.

The material 10 having a high coefficient of friction may, for instance, be a textile material, TECHFLOCK, manufactured by Lakema AB, Gothenburg, Sweden, or a metallic material, SANDPLATE, manufactured by Sandviken AB, Sandviken, Sweden.

## Claims

1. A method for producing an absorbent structure in an absorbent article (14), such as a sanitary napkin, panty guard, incontinence guard, diaper or the like, wherein a non-woven casing material (2) which is provided with one or more elastic threads or is in itself elastic is delivered in a stretched state to an air-permeable forming element (3) of a mat-forming apparatus, **characterized** by applying the elastic non-woven casing material (2) to a material (10) which has a high coefficient of friction, with which the forming element (3) is covered; depositing absorbent material (16) on the non-woven casing material (2); and removing the non-woven casing material (2) and the deposited absorbent material (16) from the forming element (3).

2. A method according to Claim 1, **characterized** in that further elements, such as rolls, rollers, conveyor belts, etc., which during the manufacturing process come into contact with the article, are also covered with a material (10) having a high coefficient of friction.

3. A method according to Claim 1 or 2, **characterized** in that the material (10) having a high coefficient of friction with which one or more of the elements involved in the manufacturing process are covered is air-permeable.

4. A method according to Claim 3, **characterized** in that the material (10) having a high coefficient of friction includes a textile material, such as needle-felt, fleece or like material whica has preferably standing fibres.

5. A method according to Claim 4, **characterized** in that the material (10) having a high coefficient of friction with which the air-permeable forming element (3) is covered includes a multiple of air-throughflow holes.

6. A method according any one of Claims 1-3, **characterized** in that the material (10) having a high coefficient of friction is comprised of strips or other discrete elements comprised of metal, ceramic, plastic or composite material having a surface which has a high coefficient of friction.

7. A method according to Claim 6, **characterized** in that the strips of material (10) having a high coefficient of friction are fastened, preferably parallel with one another, transversely to the movement direction of the absorbent articles on a flexible material with which the elements are covered where firm holding of the preferably elastic non-woven material is desired.

8. A method according to Claim 7, **characterized** in that the flexible material on which the strips of material (10) having a high coefficient of friction are fastened is air-permeable.

9. A method according to Claim 8, **characterized** in that the strips of material (10) having a high coefficient of friction are fastened to the flexible and air-permeable material in a manner to form narrow interspaces, so-called air gaps, between said strips, thereby to obtain said air-permeability.

10. An apparatus for producing an absorbent structure in an absorbent article (14), such as a sanitary napkin, panty guard, incontinence guard, diaper or the like, said apparatus comprising an air-permeable forming element (3) to which a non-woven casing material (2), which is provided with one or more elastic threads or is in itself elastic, is delivered in a stretched state and is maintained stretched during deposition of absorbent material (16) thereon, **characterized** in that the forming element (3) is covered with a material (10) which has a high coefficient of friction.

11. An apparatus according to Claim 10, **characterized** in that further elements, such as rolls, rollers, conveyor belts, etc., which during the manufacturing process come into contact with the article are alsocovered with a material (10) having a high coefficient of friction.

12. An apparatus according to Claim 10 or 11, **characterized** in that the material (10) having a high coefficient of friction with which one or more of the elements involved in the manufacturing process are covered is air-permeable.

13. An apparatus according to Claim 12, **characterized** in that the material (10) having a high coefficient of friction includes a textile material, such as needle-felt, fleece or like material which has preferably standing fibres.

14. An apparatus according to Claim 13, **characterized** in that the material (10) having a high coefficient of friction with which the air-permeable forming element (3) is covered includes a multiple of air-throughflow holes.

15. An apparatus according to any one of Claims 10-12, **characterized** in that the material (10) having a high coefficient of friction is comprised of strips or other discrete elements comprised of metal, ceramic, plastic or composite material having a surface which has a high coefficient of friction.

16. An apparatus according to Claim 15, **characterized** in that the strips of material (10) having a high coefficient of friction are fastened, preferably parallel with one another, transversely to the movement direction of the absorbent articles on a flexible material with which the elements are covered where firm holding of the preferably elastic non-woven material is desired.

17. An apparatus according to Claim 16, **characterized** in that the flexible material on which the strips of material (10) having a high coefficient of friction are fastened is air-permeable.

18. An apparatus according to Claim 17, **characterized** in that the strips of material (10) having a high coefficient of friction are fastened to the flexible and air-permeable material in a manner to form narrow interspaces, so-called air gaps, between said strips, thereby to obtain said air-permeability.

## Patentansprüche

1. Verfahren zur Herstellung einer absorbierenden Struktur in einem absorbierenden Artikel (14), wie beispielsweise eine Hygienebinde, ein Höschenschutz, ein Inkontinenzschutz, eine Windel oder dergleichen, bei dem ein mit einem oder mehreren elastischen Bändern versehenes oder selbst elastisches Deckvlies (2) in gedehntem Zustand einem luftdurchlässigen Formelement (3) einer mattenbildenden Vorrichtung zugeführt wird, **gekennzeichnet durch** Aufbringen des elastischen Deckvlieses (2) auf ein einen hohen Reibungskoeffizienten aufweisendes Material (10), mit dem das Formelement (3) bedeckt ist, Ablegen eines absorbierenden Materials (16) auf dem Deckvlies (2) und Wegnehmen des Deckvlieses (2) und des abgelegten absorbierenden Materials (16) von dem Formelement (3).

2. Verfahren nach Anspruch 1, **dadurch gekennzeichnet, daß** ferner Elemente, wie beispielsweise Rollen, Walzen, Förderbänder etc., die während des Herstellungsprozesses mit dem Artikel in Kontakt kommen, ebenfalls mit einem Material (10) beschichtet sind, das einen hohen Reibungskoeffizienten hat.

3. Verfahren nach Anspruch 1 oder 2, **dadurch gekennzeichnet, daß** das Material (10) mit hohem Reibungskoeffizienten, mit dem ein oder mehrere der Elemente, die beim Herstellungsprozeß eingesetzt werden, beschichtet sind, luftdurchlässig ist.

4. Verfahren nach Anspruch 3, **dadurch gekennzeichnet, daß** das Material (10) mit hohem Reibungskoeffizienten ein Textilmaterial enthält, wie beispielsweise ein Nadelfilz, ein Vlies oder ein ähnliches Material, das vorzugsweise aufrechtstehende Fasern umfaßt.

5. Verfahren nach Anspruch 4, **dadurch gekennzeichnet, daß** das Material (10) mit hohem Reibungskoeffizienten, mit dem das luftdurchlässige Formelement (3) bedeckt ist, eine Vielzahl von Luftdurchströmungsöffnungen enthält.

6. Verfahren nach einem der Ansprüche 1 - 3, **dadurch gekennzeichnet, daß** das Material (10) mit hohem Reibungskoeffizienten aus Streifen oder anderen einzelnen Elementen aus Metall, Keramik, Kunststoff oder einem Verbundmaterial besteht, das eine Oberfläche hat, die einen hohen Reibungskoeffizienten aufweist.

7. Verfahren nach Anspruch 6, **dadurch gekennzeichnet, daß** die Materialstreifen (10) mit hohem Reibungskoeffizienten quer zur Bewegungsrichtung der absorbierenden Artikel, vorzugsweise parallel zueinander, auf einem flexiblen Material befestigt werden, mit dem die Elemente dort bedeckt sind, wo das Festhalten des vorzugsweise elastischen Vliesmaterials erwünscht ist.

8. Verfahren nach Anspruch 7, **dadurch gekennzeichnet, daß** das flexible Material, auf welchem die Materialstreifen (10) mit hohem Reibungskoeffizienten befestigt sind, luftdurchlässig ist.

9. Verfahren nach Anspruch 8, **dadurch gekennzeichnet, daß** die Materialstreifen (10) mit hohem Reibungskoeffizienten an dem flexiblen und luftdurchlässigen Material in einer Weise befestigt sind, daß zwischen den Streifen schmale Zwischenspalte gebildet werden, sogenannte Luftspalte, um hierdurch Luftdurchlässigkeit zu erzielen.

10. Vorrichtung zur Herstellung einer absorbierenden Struktur in einem absorbierenden Artikel (14), wie beispielsweise eine Hygienebinde, ein Höschenschutz, ein Inkontinenzschutz, eine Windel oder dergleichen, wobei die Vorrichtung ein luftdurchlässiges Formelement (3) umfaßt, zu welchem ein mit einem oder mehreren elastischen Bändern versehenes oder selbst elastisches Deckvlies (2) in gedehntem Zustand zugeführt und während des Ablegens von absorbierendem Material (16) hierauf gedehnt gehalten wird, **dadurch gekennzeichnet, daß** das Formelement (3) mit einem Material (10) beschichtet ist, das einen hohen Reibungskoeffizienten aufweist.

11. Vorrichtung nach Anspruch 10, **dadurch gekennzeichnet, daß** ferner Elemente, wie beispielsweise Rollen, Walzen, Förderbänder etc., die während des Herstellungsprozesses mit dem Artikel in Kontakt treten, ebenfalls mit einem Material (10) beschichtet sind, das einen hohen Reibungskoeffizienten besitzt.

12. Vorrichtung nach Anspruch 10 oder 11, **dadurch gekennzeichnet, daß** das Material (10) mit hohem Reibungskoeffizienten, mit dem ein oder mehrere der im Herstellungsprozeß involvierten Elemente bedeckt sind, luftdurchlässig ist.

13. Vorrichtung nach Anspruch 12, **dadurch gekennzeichnet, daß** das Material (10) mit hohem Reibungskoeffizienten ein Textilmaterial enthält, wie beispielsweise ein Nadelfilz, Vlies oder ein ähnliches Material, das vorzugsweise aufrechtstehende Fasern besitzt.

14. Vorrichtung nach Anspruch 13, **dadurch gekennzeichnet, daß** das Material (10) mit hohem Reibungskoeffizienten, mit dem das luftdurchlässige Formelement (3) bedeckt ist, eine Vielzahl Luftdurchströmungslöcher enthält.

15. Vorrichtung nach einem der Ansprüche 10 - 12, **dadurch gekennzeichnet, daß** das Material (10) mit hohem Reibungskoeffizienten aus Streifen oder anderen einzelnen Elementen aus Metall, Keramik, Kunststoff oder einem Verbundmaterial besteht, das eine Oberfläche hat, die einen hohen Reibungskoeffizienten aufweist.

16. Vorrichtung nach Anspruch 15, **dadurch gekennzeichnet, daß** die Materialstreifen (10) mit hohem Reibungskoeffizienten quer zur Bewegungsrichtung der absorbierenden Artikel, vorzugsweise parallel zueinander, auf einem flexiblen Material befestigt sind, mit dem die Elemente dort bedeckt sind, wo ein Festhalten des vorzugsweise elastischen Vliesmaterials erwünscht ist.

17. Vorrichtung nach Anspruch 16, **dadurch gekennzeichnet, daß** das flexible Material, auf dem die Materialstreifen (10) mit hohem Reibungskoeffizienten befestigt sind, luftdurchlässig ist.

18. Vorrichtung nach Anspruch 17, **dadurch gekennzeichnet, daß** die Materialstreifen (10) mit hohem Reibungskoeffizienten an dem flexiblen und luftdurchlässigen Material derart befestigt sind, daß zwischen den Streifen schmale Zwischenspalte gebildet sind, sogenannte Luftspalte, um hierdurch Luftdurchlässigkeit zu erzielen.

## Revendications

1. Procédé pour produire une structure absorbante dans un article absorbant (14), tel qu'un change hygiénique, une culotte de propreté, une protection contre l'incontinence, une couche-culotte ou autre article analogue, dans lequel un matériau non-tissé (2) formant enveloppe, qui est pourvu d'un ou plusieurs fils élastiques ou est lui-même élastique, est délivré dans un état étiré à un élément de formation (3) perméable à l'air d'un appareil de formation de mat, caractérisé en que l'on applique le matériau non-tissé (2) formant enveloppe à un matériau (10) qui a un coefficient de frottement élevé et avec lequel l'élément de formation (3) est recouvert; on dépose un matériau absorbant (16) sur le matériau non-tissé (2) formant enveloppe; et on retire de l'élément de formation (3) le matériau non-tissé (2) formant enveloppe et le matériau absorbant déposé (16).

2. Procédé selon la revendication 1, caractérisé en ce que d'autres éléments, tels que des cylindres, des rouleaux, des courroies transporteuses, etc, qui, pendant l'opération de fabrication viennent en contact avec l'article, sont aussi recouverts d'un matériau (10) ayant un coefficient de frottement élevé.

3. Procédé selon la revendication 1 ou 2, caractérisé en ce que le matériau (10) ayant un coefficient de frottement élevé et avec lequel sont recouverts un ou plusieurs éléments impliqués dans l'opération de fabrication est perméable à l'air.

4. Procédé selon la revendication 3, caractérisé en ce que le matériau (10) ayant un coefficient de frottement élevé comprend un matériau textile, tel qu'un feutre aiguilleté, un molleton ou autre matériau analogue qui comporte de préférence des fibres fixes.

5. Procédé selon la revendication 4, caractérisé en ce que le matériau (10) ayant un coefficient de frottement élevé et avec lequel est recouvert l'élément de formation (3) perméable à l'air comprend de multiple trous laissant passer l'air.

6. Procédé selon l'une quelconque des revendications 1-3, caractérisé en ce que le matériau (10) ayant un coefficient de frottement élevé est constitué de bandes ou autres éléments discrets constitués de métal, de céramique, de matière plastique ou de matière composite ayant une surface qui présente un coefficient de frottement élevé.

7. Procédé selon la revendication 6, caractérisé en ce que les bandes de matériau (10) ayant un coefficient de frottement élevé sont fixées, de préférence les unes aux autres, transversalement à la direction de déplacement des articles absorbants sur un matériau flexible avec lequel les éléments sont recouverts si on désire un maintien ferme du matériau non-tissé de préférence élastique.

8. Procédé selon la revendication 7, caractérisé en ce que le matériau flexible sur lequel sont fixées les bandes de matériau (10) ayant un coefficient de frottement élevé est perméable à l'air.

9. Procédé selon la revendication 8, caractérisé en ce que les bandes de matériau (10) ayant un coefficient de frottement élevé sont fixées sur le matériau flexible et perméable à l'air de manière à former des intervalles étroits, ce que l'on appelle des interstices, entre lesdites bandes afin d'obtenir ainsi ladite perméabilité à l'air.

10. Appareil pour produire une structure absorbante dans un article absorbant (14) tel qu'un change hygiénique, une culotte de propreté, une protection contre l'incontinence, une couche-culotte, ou autre article analogue, ledit appareil comprenant un élément de formation (3) perméable à l'air, auquel un matériau non-tissé (2) formant enveloppe, qui est pourvu d'un ou plusieurs fils élastiques ou est lui-même élastique, est délivré dans un état étiré et est maintenu étiré pendant qu'y est déposé un matériau absorbant (16), caractérisé en ce que l'élément de formation (3) est recouvert d'un matériau (10) qui a un coefficient de frottement élevé.

11. Appareil selon la revendication 10, caractérisé en ce que d'autres éléments, tels que des cylindres, des rouleaux, des courroies transporteuses, etc., qui, pendant l'opération de fabrication, viennent en contact avec l'article sont aussi recouverts d'un matériau (10) ayant un coefficient de frottement élevé;

12. Appareil selon la revendication 10 ou 11, caractérisé en ce que le matériau (10) ayant un coefficient de frottement élevé et avec lequel sont recouverts un ou plusieurs éléments impliqués dans l'opération de fabrication est perméable à l'air.

13. Appareil selon la revendication 12, caractérisé en ce que le matériau (10) ayant un coefficient de frottement élevé comprend un matériau textile, tel qu'un feutre aiguilleté, un molleton ou autre matériau analogue qui comporte de préférence des fibres fixes.

14. Appareil selon la revendication 13, caractérisé en ce que le matériau (10) ayant un coefficient de frottement élevé et avec lequel est recouvert l'élément de formation (3) perméable à l'air comprend de multiple trous laissant passer l'air.

15. Appareil selon l'une quelconque des revendications 10-12, caractérisé en ce que le matériau (10) ayant un coefficient de frottement élevé est constitué de bandes ou autres éléments discrets constitués de métal, de céramique, de matière plastique ou de matière composite ayant une surface qui présente un coefficient de frottement élevé.

16. Appareil selon la revendication 15, caractérisé en ce que les bandes de matériau (10) ayant un coefficient de frottement élevé sont fixées, de préférence les unes aux autres, transversalement à la direction de déplacement des articles absorbants, sur un matériau flexible avec lequel les éléments sont recouverts si on désire un maintien ferme du matériau non-tissé de préférence élastique.

17. Appareil selon la revendication 16, caractérisé en ce que le matériau flexible sur lequel sont fixées les bandes de matériau (10) ayant un coefficient de frottement élevé est perméable à l'air.

18. Appareil selon la revendication 17, caractérisé en ce que les bandes de matériau (10) ayant un coefficient élevé de frottement sont fixées sur le matériau flexible et perméable à l'air de manière à former des intervalles étroits, ce que l'on appelle des interstices, entre lesdites bandes afin d'obtenir ainsi ladite perméabilité à l'air.
